(19) Europäisches Patentamt
European Patent Office
Office européen des brevets

(11) **EP 3 161 779 B1**

(12) **EUROPEAN PATENT SPECIFICATION**

(45) Date of publication and mention of the grant of the patent:
**26.07.2023 Bulletin 2023/30**

(21) Application number: **15815912.9**

(22) Date of filing: **29.06.2015**

(51) International Patent Classification (IPC):
***G06Q 50/22*** *(2018.01)*

(52) Cooperative Patent Classification (CPC):
**A61B 5/1123; A61B 5/1118; A61B 5/4809; A61B 5/681;** A61B 5/1112; A61B 5/7278; A61B 2560/0242

(86) International application number:
**PCT/US2015/038289**

(87) International publication number:
**WO 2016/003887 (07.01.2016 Gazette 2016/01)**

(54) **AUTOMATIC RESET OF PHYSICAL PERFORMANCE INFORMATION**

AUTOMATISCHES ZURÜCKSETZEN VON KÖRPERLICHEN LEISTUNGSINFORMATIONEN

RESTAURATION AUTOMATIQUE D'INFORMATIONS DE PERFORMANCE PHYSIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2014 US 201462018922 P**
**26.06.2015 US 201514751248**

(43) Date of publication of application:
**03.05.2017 Bulletin 2017/18**

(73) Proprietor: **Garmin Switzerland GmbH**
**8200 Schaffhausen (CH)**

(72) Inventor: **LAUGHLIN, Daniel B.**
**Overland Park, KS 66207 (US)**

(74) Representative: **Bird & Bird LLP**
**Am Sandtorkai 50**
**20457 Hamburg (DE)**

(56) References cited:
EP-A1- 1 770 369          WO-A1-2006/003243
WO-A1-2013/069002     CN-U- 203 274 757
KR-A- 20110 011 195     US-A1- 2010 079 294
US-A1- 2012 143 095     US-A1- 2014 009 258
US-B1- 8 734 296

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND

Users of fitness monitoring devices commonly wish to track and view their physical activity over some period of time. Conventional fitness monitoring devices may determine and store physical performance information for a monitoring period (e.g., a 24-hour period).

For instance, some devices may determine and store physical performance information for one day and present this information on a display to a user so the user can gauge his progress for that day.

At the end of the monitoring period, the physical performance information may be reset, allowing the user to gauge his progress for the next monitoring period, and so on.

Conventional fitness monitoring devices typically reset the physical performance information after this monitoring period has completed at a predetermined time, such as midnight, or using a manual reset, which may be inconvenient for the user or be forgotten.

Even when the reset function is performed automatically, conventional fitness monitoring devices must have knowledge of the current time to accurately perform this reset function upon the expiration of the monitoring period. As a result, current techniques of resetting fitness monitoring periods have several drawbacks.

WO 2013/069002 A1 discloses a monitoring system adapted to detect and prevent a risk of venous pooling. A sensor worn by a user, such as a sensor measuring acceleration and tilt of a limb segment, and a processor are adapted to determine user posture data and, in particular, to detect if a user exceeds a maximum allowable time for maintaining a static upright posture without changing to a different posture or commencing activity. For this purpose, a timer is started upon detection of an upright posture, and if the maximum allowable time is exceeded without detecting a change to a different posture of or commencement of activity the system alerts the user to the risk of venous pooling.

US 2010/079294 A1 discloses an apparatus for estimating a living being's alertness based on signals from sensors, such as an activity sensor, from which signals transitions between a sleep cycle and a wake cycle are determined.

WO 2006/003243 A1 discloses a system for monitoring a person on a base. In particular, it discloses a system which resets a timer every time the movements of a patient lying down in a bed exceed a threshold. If the timer reaches a predetermined time, such as e.g. two hours, an alarm is issued to inform a nurse that the patient is threatened by a bed sour as a result of long inactivity.

US 2012/143095 A1 discloses a system for monitoring activity of a person, with means to reset time when certain motion conditions (sleep after certain activity period) is detected.

## SUMMARY

Embodiments of the present technology relate generally to a fitness monitoring device and, more particularly, to a fitness monitoring device that performs an automatic reset of a monitoring period over which physical performance information is determined and stored without having accurate knowledge of the current time. A fitness monitoring device that is worn by a user who has not manually input a current time (or time zone) or connected the device to a computing device in order to obtain a current time may desire to reset the physical performance information at approximately midnight or when the user is asleep. Alternatively, the user may have traveled to a different time zone during the predetermined time period over which physical performance is determined and stored and the user may desire to reset the physical performance information at a time period that is different than the predetermined time period to account for the new time zone

In embodiments, a fitness-monitoring device may include a sensor configured to measure movements of a user wearing the fitness monitoring device and to generate motion data, a memory configured to store the motion data and physical performance information; and a processor configured to determine the physical performance information based on the motion data, track the physical performance information over a predetermined period of time, calculate, utilizing the motion data, a reset time when a period of inactivity within each of a plurality of consecutive sampling periods exceeds a threshold time period; and reset the tracked physical performance information to zero at the reset time to restart tracking of the physical performance information for an approximate one-day period.

The fitness monitoring device may track a user's physical performance, such as a number of steps taken by the user over a predetermined time period, and display the user's progress towards reaching a performance goal, which may represent a threshold number of steps to be reached by the user over a one-day period. The predetermined time period may represent any suitable time period, such as less than twenty-four hours or a full day. The fitness monitoring device may initially reset the physical performance information when a period of inactivity within each of a plurality of consecutive sampling periods exceeds a threshold time period to reset tracking of the physical performance information for an approximate one-day period of twenty-four hours. For instance, the fitness monitoring device may reset the physical performance information when the user is determined to likely be asleep and then determine, utilizing motion data stored in a memory over an extended period of time, an approximated midnight time and adjust the reset time to equal the approximated midnight time in order for the reset of physical performance information to occur every day at approximately midnight. Embodiments are described to automatically reset the tracked physical performance information without manual input or requiring knowledge of the current time or time zone. In embodiments, the sampling period may be one hour, the predetermined period of time may be less than twenty-four hours and the extended period of time may be four days.

The fitness monitoring device may determine a user's sleep time and utilize this time as a reset time by which to reset the tracked physical performance information associated with the user before he went to sleep. In some embodiments, motion data generated by the fitness monitoring device may be sampled over several consecutive sampling periods to determine whether the user is likely asleep. In other embodiments, light intensity data generated by the fitness monitoring device based on the environment of the fitness monitoring device may be sampled over several consecutive sampling periods to determine whether the fitness monitoring device is located in a location having low levels of ambient light for an extended period of time, which is likely associated with the user being asleep. Once the reset time is established, the fitness monitoring device may use the reset time to automatically reset the tracked physical performance information according to a recurring schedule (e.g., once per day if physical performance information is being tracked daily) to reset the physical performance information each day at this same reset time. The reset time may be used as part of a recurring schedule in this way without knowledge of and/or independent of the current time of day.

This summary is provided to introduce a selection of concepts in a simplified form that are further described below in the detailed description. This summary is not intended to identify key features or essential features of the claimed subject matter, nor is it intended to be used to limit the scope of the claimed subject matter. Other aspects and advantages of the present technology will be apparent from the following detailed description of the embodiments and the accompanying drawing figures.

BRIEF DESCRIPTION OF THE DRAWINGS

The figures described below depict various aspects of the system and methods disclosed herein. It should be understood that each figure depicts an embodiment of a particular aspect of the disclosed system and methods, and that each of the figures is intended to accord with a possible embodiment thereof. Further, whenever possible, the following description refers to the reference numerals included in the following figures, in which features depicted in multiple figures are designated with consistent reference numerals.

FIG. 1 is an illustration of a block diagram of an exemplary fitness monitoring system 100 in accordance with an embodiment of the present disclosure;

FIG. 2A is an illustration of exemplary consecutive data sampling periods 200 for calculating a reset time, according to an embodiment;

FIG. 2B is an illustration of exemplary single data sampling period 250 for calculating a reset time, according to an embodiment;

FIG. 3 is an illustration of an exemplary fitness monitoring device 300 in accordance with an embodiment of the present disclosure;

FIG. 4 is an illustration of an exemplary fitness monitoring device 400 in accordance with an embodiment of the present disclosure; and

FIG. 5 illustrates a method flow 500, according to an embodiment.

DETAILED DESCRIPTION

The following text sets forth a detailed description of numerous different embodiments. However, it should be understood that the detailed description is to be construed as exemplary only and does not describe every possible embodiment since describing every possible embodiment would be impractical. In light of the teachings and disclosures herein, numerous alternative embodiments may be implemented.

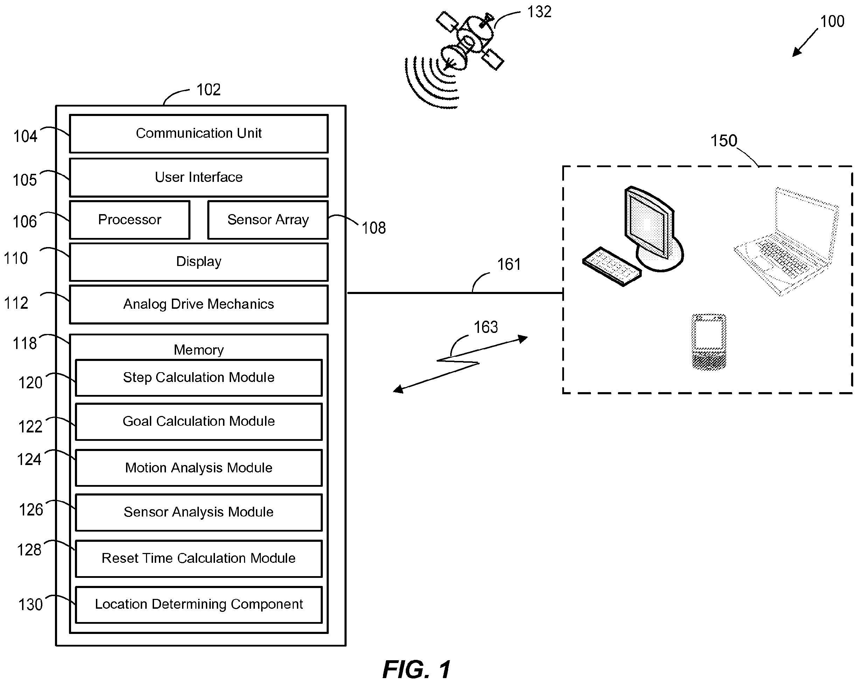

FIG. 1 is an illustration of a block diagram of an exemplary fitness monitoring system 100 in accordance with an embodiment of the present disclosure. In some embodiments, fitness monitoring device 102 may act as a standalone device and not require communications with external computing devices 150. But in other embodiments, which are further discussed below, fitness monitoring device 102 may communicate with and/or work in conjunction with one or more of external computing devices 150, which may provide a current time to the fitness monitoring device 102. In accordance with such embodiments, fitness monitoring device 102 and one or more external computing devices 150 may be configured to communicate with one another using any suitable number of wired and/or wireless links (e.g., wired link 161 and/or wireless link 163) in conjunction with any suitable number and type of communication protocols.

In an embodiment, one or more of external computing devices 150 may include any suitable number and/or type of computing devices configured to facilitate user interaction and data exchange with fitness monitoring device 102. For example, one or more of external computing devices 150 may be implemented as a mobile computing device (e.g., smartphone, tablet, laptop, phablet, netbook, notebook, pager, personal digital assistant (PDA), wearable computing device, smart glasses, a smart watch or a bracelet, etc.), or any other suitable type of computing device capable of wired and/or wireless communication (e.g., a desktop computer).

In an embodiment, one or more of external computing devices 150 may facilitate user interaction such as, for example, viewing various types of fitness data, such as motion data and physical performance information generated and/or stored by fitness monitoring device 102. The fitness data may include, for example, physical performance information, such as a number of steps taken by a user within a period of time *(e.g.,* a one-day period), and a threshold physical performance, such as a number of steps associated with a fitness activity goal. The fitness monitoring device 102 may also store and provide a history of fitness activity goals and/or physical performance information, calculated reset times, adjustments to reset times, location (e.g., GPS) coordinates, and a current time-of-day, etc. Communications between one or more of external computing devices 150 and fitness monitoring device 102 may facilitate the transfer of data between these devices.

In another embodiment, fitness monitoring device 102 need not communicate with one or more of external computing devices 150 to generate, store, and/or display the various types of fitness data. As will be further discussed below, fitness monitoring device 102 may operate as a standalone device to maintain and/or display the time-of-day, to generate motion data and/or other sensory data, to track physical performance information (e.g., a number of steps taken) associated with a user wearing fitness monitoring device 102, to display the physical performance information collected (e.g., number of steps taken) over a period of time *(e.g.,* a one-day period), to calculate a reset time, to adjust a reset time, and to perform a reset of the tracked physical performance information.

Fitness monitoring device 102 may be implemented as any suitable type of portable, mobile, and/or wearable device configured to measure and/or provide user activity in accordance with the embodiments described herein. For example, fitness monitoring device 100 may be implemented as an analog watch or a digital watch worn by a user. To provide additional examples, fitness monitoring device 100 may be implemented as any suitable type of device having a suitable display (*e.g.,* a liquid crystal display (LCD), a light-emitting diode (LED) display, etc.) to show user activity, such as a digital watch, a worn electronic device, a mobile computing device *(e.g.,* a smartphone) a wrist or ankle bracelet, etc.

In an embodiment, fitness monitoring device 102 may include a communication unit 104, a location determining component 130, a user interface 105, a processor 106, a sensor array 108, a display 110, analog drive mechanics 112, and a memory 118. Fitness monitoring device 102 may include additional elements such as, for example, power sources, motor controllers, ports, interconnects, etc., which are not described herein for purposes of brevity. Fitness monitoring device 102 may include more or less elements based upon the implementation of fitness monitoring device 102. For example, if implemented as a digital device, fitness monitoring device 102 may not include analog drive mechanics 112.

To provide another example, if implemented as an analog watch, fitness monitoring device 102 may not include display 110.

Communication unit 104 may be configured to support any suitable number and/or type of communication protocols to facilitate communications between fitness monitoring device 102 and one or more of external computing devices 150. Communication unit 104 may be configured to receive any suitable type of information via one or more of external computing devices 150, which may be displayed on fitness monitoring device 102 in various ways and is further discussed below. Communication unit 104 may be implemented with any suitable combination of hardware and/or software to facilitate this functionality. For example, communication unit 104 may be implemented with any number of wired and/or wireless transceivers, ports, connectors, antennas, etc.

Communication unit 104 may be configured to facilitate communications with various external computing devices 150 using different types of communication protocols. For example, communication unit 104 may communicate with a mobile computing device via a wireless BLUETOOTH communication protocol (e.g., via wireless link 163) and with a laptop or a personal computer via a wired universal serial bus (USB) protocol (e.g., via wired link 161). Communication unit 104 may be configured to support simultaneous or separate communications between two or more of external computing devices 150.

The location determining component 130 may be a satellite navigation receiver that works with a global navigation satellite system (GNSS) such as the global positioning system (GPS) primarily used in the United States, the GLONASS system primarily used in the Soviet Union, the BeiDou system primarily used in China, or the Galileo system primarily used in Europe. The GNSS includes a plurality of satellites 132 in orbit about the Earth. The orbit of each satellite is not necessarily synchronous with the orbits of other satellites and, in fact, is likely asynchronous. A GNSS equipped device such as the fitness monitoring device 102 is shown receiving spread spectrum satellite signals from the various satellites 132. The spread spectrum signals continuously transmitted from each satellite use a highly accurate frequency standard accomplished with an extremely accurate atomic clock. Each satellite 132, as part of its data signal transmission, transmits a data stream indicative of that particular satellite. The fitness monitoring device 102 must acquire spread spectrum satellite signals from at least three satellites 132 for the receiver device to calculate its two-dimensional position by triangulation. Acquisition of an additional signal, resulting in signals from a total of four satellites 132, permits the fitness monitoring device 102 to calculate its three-dimensional position.

The location determining component 130 and the processor 106 are operable to receive navigational signals from the satellites 132 and to calculate positions of the fitness monitoring device 102 as a function of the signals. The location determining component 130 and processor 106 may also determine track logs or any other series of geographic coordinates corresponding to points along a route or other path traveled by a user of the fitness monitoring device 102. The location determining component 130 and/or the processor 106 may also be operable to calculate routes to desired locations, provide instructions to navigate to the desired locations, display maps and other information on the display screen, and to execute other functions described herein.

The location determining component 130 may include one or more processors, controllers, or other computing devices and memory so that it may calculate location and other geographic information without the processor 106 or it may utilize the components of the processor 106. Further, the location determining component 130 may be integral with the processor 106 such that the location determining component 130 may be operable to specifically perform the various functions described herein. Thus, the processor 106 and location determining component 130 can be combined or be separate or otherwise discrete elements.

The location determining component 130 may include an antenna to assist in receiving the satellite signals. The antenna may be a patch antenna, a linear antenna, or any other type of antenna that can be used with navigational devices. The antenna may be mounted directly on or in the housing or may be mounted external to the housing.

Although embodiments of the fitness monitoring device 102 may include a satellite navigation receiver, it will be appreciated that other location-determining technology may be used. For example, the communication unit 104 may be used to determine the location of the fitness monitoring device 102 by receiving data from at least three transmitting locations and then performing basic triangulation calculations to determine the relative position of the device with respect to the transmitting locations. For example, cellular towers or any customized transmitting radio frequency towers can be used instead of satellites 132. With such a configuration, any standard geometric triangulation algorithm can be used to determine the location of the fitness monitoring device 102.

In other embodiments, the location determining component 130 need not directly determine the current geographic location of the fitness monitoring device 102. For instance, the location determining component 130 may determine the current geographic location through a communications network, such as by using Assisted Global Positioning System (A-GPS) by receiving communications from a combination of base stations and/or satellites 132, or from another electronic device. The location determining component 130 may even receive location data directly from a user. For example, a user may obtain location data for a physical activity before and after it has been completed from another satellite navigation receiver or from another source and then manually input the data into the fitness monitoring device 102.

Although fitness monitoring device 102 may utilize location determining component 130 to determine a geographic location by determining coordinates associated with a current position, the Coordinated Universal Time (UTC) does not provide a current time unless additional information, such as a table associating coordinates with their respective time zone, is available for reference.

User interface 105 may be configured to facilitate user interaction with fitness monitoring device 102. For example, user interface 105 may include a user-input device such as an interactive portion of display 110 (*e.g.,* a "soft" keyboard and/or buttons displayed on display 110), physical buttons integrated as part of fitness monitoring device 102 that may have dedicated and/or multi-purpose functionality, one or more watch crowns (if fitness monitoring device 102 is implemented as a watch), etc.

Sensor array 108 may be implemented as any suitable number and/or type of sensors configured to measure, monitor, and/or quantify a user's motion while wearing fitness monitoring device 102 as motion data. Sensor array 108 may be advantageously mounted or otherwise positioned within fitness monitoring device 102 to facilitate these functions. Sensor array 108 may be configured to measure a user's motion and/or to generate motion data continuously, or in accordance with any suitable recurring schedule, such as, for example, once per every 5 seconds, once per every 10 seconds, once per every 30 seconds, once per minute, etc.

Examples of suitable sensor types implemented by sensor array 108 may include one or more accelerometers, gyroscopes, perspiration detectors, compasses, speedometers, magnetometers, barometers, thermometers, proximity sensors, light sensors (*e.g.*, light intensity detectors), photodetectors, photoresistors, photodiodes, Hall Effect sensors, electromagnetic radiation sensors (*e.g.*, infrared and/or ultraviolet radiation sensors), humistors, hygrometers, altimeters, biometrics sensors (*e.g.*, heart rate monitors, blood pressure monitors, skin temperature monitors), microphones, etc.

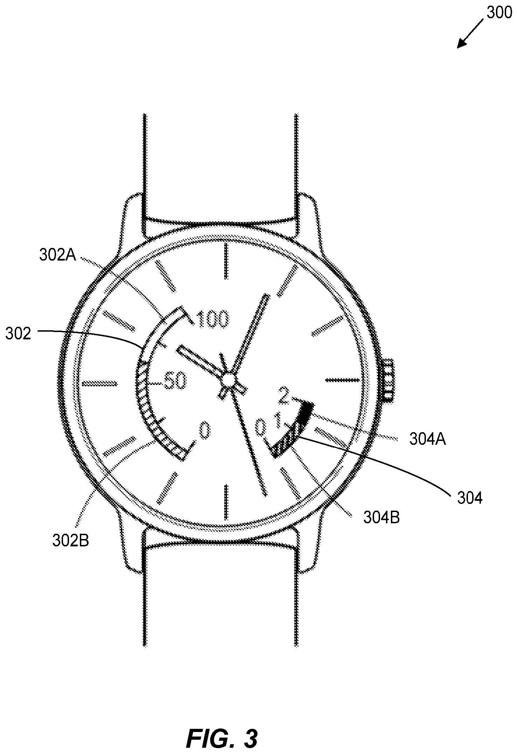

Analog drive mechanics 112 may be configured as any suitable number and/or type of motors (*e.g.*, stepper motors, synchronous motors, impulse motors, direct current (DC) motors, off-the-shelf watch motors, servos), quarts movement clocks, etc., to facilitate implementations of fitness monitoring device 102 as an analog watch. For example, analog drive mechanics 112 may function to, for example, provide quartz movement of watch components such as second, minute, and hour hands and/or to rotate discs to provide a gauge of user activity and inactivity, which is further discussed below with reference to FIG. 3.

Processor 106 may be implemented as any suitable type and/or number of processors, such as a host processor of fitness monitoring device 102, for example. To provide additional examples, processor 106 may be implemented as an application specific integrated circuit (ASIC), an embedded processor, a central processing unit associated with fitness monitoring device 102, etc. Processor 106 may be configured to communicate with one or more of communication unit 104, user interface 105, sensor array 108, analog drive mechanics, and/or memory 118 via one or more wired and/or wireless interconnections, such as any suitable number of data and/or address buses, for example. These interconnections are not shown in FIG. 1 for purposes of brevity. Processor 106 may be configured to operate in conjunction with one or more of one or more of communication unit 104, user interface 105, sensor array 108, analog drive mechanics 112, and/or memory 118 to process data, to store data to memory 118, to retrieve data from memory 118, to display information on display 110, to move one or more portions of analog drive mechanics 112, to receive, process, and/or interpret data from sensor array 108 such as motion data, sensor data (e.g., light intensity data), and/or user interactions, to receive data from one or more of external computing devices 150, etc.

In accordance with various embodiments, memory 118 may be a computer-readable non-transitory storage device that may include any suitable combination of volatile (e.g., a random access memory (RAM), or non-volatile memory (e.g., battery-backed RAM, FLASH, etc.). Memory 118 may be configured to store instructions executable on processor 106, such as the various memory modules illustrated in FIG. 1 and further discussed below, for example. These instructions may include machine readable instructions that, when executed by processor 106, cause processor 106 to perform various acts as described herein. Memory 118 may also be configured to store any other suitable data, such as data received from one or more of external computing devices 150 via communication unit 104, data measured, calculated, and/or generated via sensor array 108, fitness activity goals, data indicative of user activity and/or user inactivity over a period of time (e.g., daily logs of activity and/or inactivity data), etc.

Memory 118 may include a module to determine and store physical performance information. In embodiments, step calculation module 120 is a region of memory 118 configured to store instructions, that when executed by processor 106, cause processor 106 to perform various acts in accordance with applicable embodiments as described herein. Although referred to herein as "steps," step calculation module 120 may include instructions to facilitate processor 106 calculating a number of steps based upon any suitable repetitive physical performance activity, such as a user walking, running, cycling, swimming, etc.

Processor 106 may determine physical performance information based on the motion data from sensor array 108 and track physical performance information over a period of time, such as a one-day period. Processor 106 may initially reset the tracked physical performance information to zero when a period of inactivity within each of a plurality of consecutive sampling periods exceeds a threshold time period (i.e., at a reset time) to restart tracking the physical performance information for an approximate one-day period. In embodiments, processor 106 may determine, utilizing motion data stored over an extended period of time, an approximated midnight time and adjust the reset time to equal the approximated midnight time. Fitness-monitoring device 102 may store in memory 118 motion data for an extended period of time (e.g., 2 days, 4 days, 1 week, etc.) and processor 106 may utilize the stored motion data to adjust the reset time to correspond to a time approximated to be midnight for periods of time after the initial reset of physical performance information in order for subsequent resets to be more accurate. For instance, an initial reset may occur within a predetermined time period that is less than twenty-four hours because the user began using the fitness-monitoring device 102 at a time that was different than midnight, such as 2:00 P.M. in the afternoon, and the fitness-monitoring device 102 may utilize the motion data over an extended period of time to "fine tune" the reset time as additional data is analyzed, thereby continually improving its accuracy.

In an embodiment, step calculation module 122 includes instructions that, when executed by processor 106, cause processor 106 to monitor, track, measure, and/or calculate a user's steps while wearing fitness monitoring 102. For example, processor 106 may execute instructions stored in step calculation module 120 to process motion data generated by sensor array 108 and use this data to determine and track a number of steps taken by the user.

Step calculation module 120 may include instructions to facilitate processor 106 calculating a number of steps in accordance with any suitable techniques based upon the particular type and/or number of sensors implemented by sensor array 108. For example, sensor array 108 may include a three-axis accelerometer. In such a case, step calculation module 120 may include instructions to facilitate processor 106 calculating a number of steps based upon motion data generated by sensor array 108 in accordance with x, y, and z-axis accelerometer metrics.

Upon calculating a number of steps, processor 106 may execute instructions stored in step calculation module 120 to store the number of steps or other physical performance information in any suitable region of memory 118. The number of steps may be stored in memory 118 as the steps are calculated or at a subsequent time after the steps are calculated, such as a period of time after the user has stopped moving, a predetermined time after the steps are calculated, etc.

In various embodiments, processor 106 may store the number of steps in a suitable region of memory 118 correlated with other data such as a time or day when the steps were taken, a frequency of the steps over one or more time periods, a time of day when the daily fitness activity goal was achieved, a time when the daily fitness activity goal was exceeded, the corresponding proportion of the fitness activity goal that was exceeded, etc.

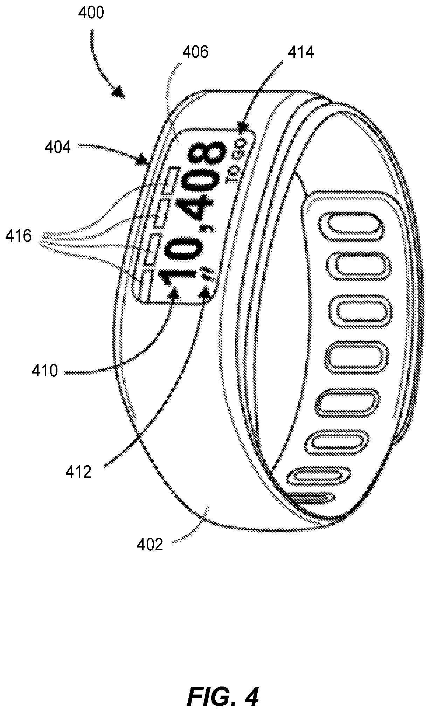

In some embodiments, upon storing the calculated number of steps and/or data associated therewith in a suitable region of memory 118, processor 106 may cause display 110 to indicate the calculated number of steps towards achieving the fitness activity goal, which is further discussed below with reference to FIG. 4. In other embodiments, upon storing the calculated number of steps and/or data associated therewith in a suitable region of memory 118, processor 106 may cause one or more portions of analog drive mechanics 112 to move to indicate the user's progress towards achieving the fitness activity goal, which is further discussed below with reference to FIG. 3.

Goal calculation module 122 is a region of memory 118 configured to store instructions, that when executed by processor 106, cause processor 106 to perform various acts in accordance with applicable embodiments as described herein. In an embodiment, goal calculation module 122 includes instructions that, when executed by processor 106, cause processor 106 to determine a fitness activity goal for a certain period of time. For example, the fitness activity goal may be a threshold physical performance, such as a number of steps to be taken by a user wearing fitness monitoring device 102, over any period of time (e.g., one day period, a two day period, a one week period, etc.).

In some embodiments, processor 106 may determine the fitness activity goal independently without communicating or receiving data from external computing devices 150. In accordance with such embodiments, goal calculation module 122 may include instructions specifying a preset, preprogrammed, and/or default fitness activity goal, which may be a threshold number of steps. Processor 106 may read these instructions to determine an initial fitness activity goal, which may remain the same over time, be increased incrementally based upon a user regularly achieving the fitness activity goal, be decreased incrementally based upon a user regularly failing to achieve the fitness activity goal, etc.

To provide an illustrative example, goal calculation module 122 may include instructions specifying an initial fitness activity goal of M steps in a one day period. This number of steps M may be increased over time, for example, when processor 106 determines that a number of days have elapsed in which the daily fitness activity goal has been achieved. In such a case, instructions stored in goal calculation module 122 may cause processor 106 to adjust the number of steps to increase M by 5%, by 10%, etc.

Again, the fitness activity goal corresponds to any threshold physical performance, such as a threshold number of steps to be taken by a user wearing fitness monitoring device 102 over a period of time, which may be a one-day period, for example. In such a case, fitness monitoring device 102 may track the number of steps taken by the user over a one-day time period (e.g., via execution of instructions stored in step calculation module 120 via processor 106). Upon expiration of the one-day time period (or other period of time associated with the fitness activity goal) the step count resets, and the user enters a new monitoring period.

During the new monitoring period, the user's steps are once again calculated, stored, and/or displayed as the user once again works towards achieving the fitness activity goal, which may remain the same, be incremented, or be decremented, as previously discussed. In conventional fitness monitoring devices, the reset time, or the time in which the new monitoring period starts, is typically manually entered or synchronized with another clock, such as a clock utilized to keep track of the time-of-day, a time received from an external device, etc. The reset time for such conventional devices may be a time commonly associated with the end of the day when a user is likely sleeping, such as midnight, for example.

However, it may be desirable for people to begin using fitness monitoring device 102 without first setting the time or connecting to another device. If a user were to do this with a conventional fitness monitoring device, the reset time would be out of sync with the activity goal period until the user somehow sets the fitness monitoring device to the correct time of day. Therefore, embodiments include fitness monitoring device 102 automatically calculating the reset time based upon an analysis of data generated via sensor array 108, as further discussed below.

Motion analysis module 124 is a region of memory 118 configured to store instructions, that when executed by processor 106, cause processor 106 to perform various acts in accordance with applicable embodiments as described herein. In an embodiment, motion analysis module 124 includes instructions that, when executed by processor 106, cause processor 106 to analyze motion data generated by sensor array 108 over various sampling intervals. Embodiments include processor 106 determining a reset time based upon the sampled motion data, which is further discussed below with reference to the instructions stored in reset time calculation module 128.

For example, using the example of a daily fitness activity goal, motion analysis module 124 may include instructions that, when executed by processor 106, cause processor 106 to sample motion data generated by sensor array 108 over several sampling intervals, which may be any suitable time interval (e.g., 30 seconds, one minute, 5 minutes, etc.).

Processor 106 may determine, based upon physical performance information, such as the number of steps indicated by the motion data within each sampling interval, whether the user was inactive (e.g., no steps taken during the sampled interval) or active (e.g., some number of steps taken during the sampled interval greater than zero). As a result of this determination, embodiments include processor 106 categorizing each sampled interval as being an inactive interval or an active interval. Processor 106 may additionally sub-categorize active intervals by an intensity level indicated by the number of steps taken within each respective interval.

Processor 106 may sub-categorize a period of moderate activity corresponding to an aggregation of time intervals over which the motion data exceeds a predetermined inactivity level and a period of strenuous activity corresponding to an aggregation of time intervals over which the motion data exceed a predetermined inactivity level and a strenuous activity threshold. For example, processor 106 may sub-categorize an active sampling interval as a moderate activity sampling interval (*e.g.*, walking) when the number of steps within a sampling interval exceeds some threshold number N1, which may be zero steps. Continuing this example, processor 106 may sub-categorize an active sampling interval as a high activity sampling interval (*e.g.*, running) when the number of steps exceeds another threshold number N2 that is greater than N1.

A sampling period may be one hour and a predetermined period of time for tracking physical performance information based on motion data from sensor array 108 may be less than twenty-four hours. In an embodiment, processor 106 may continue the process of categorizing each sampling interval as being an inactive interval or an active interval for a number of sampling intervals for an entire sampling period. A sampling period may be any suitable period of time that is made up of several sampling intervals, such as a 30 minute sampling period including 30 one-minute sampling intervals, a one-hour sampling period including 60 one-minute sampling intervals, etc.

To differentiate between a user wearing fitness monitoring device 102 being sedentary as opposed to being asleep (the latter of which being used to accurately determine a reset time) embodiments include processor 106 calculating how many sampling intervals within a sampling period are categorized as inactive intervals. Continuing the example of one-minute sampling intervals constituting a one-hour sampling period, this would translate into processor 106 determining a number of minutes over a one-hour sampling period in which a user was inactive (*e.g.,* no steps being taken).

Although a long period of inactivity over a single sampling period may be indicative of a user sleeping, this may also be the result of a user being sedentary for a temporary period of time throughout the day (*e.g.*, sitting at a desk working, watching a movie, etc.). Therefore, embodiments include processor 106 executing instructions stored in motion analysis module 124 to calculate a reset time as the start of the first of the plurality of consecutive sampling periods in which motion data based on information from sensor array 108 is below a predetermined inactivity level for a threshold time period. For instance, processor 106 may execute instructions stored in motion analysis module 124 to determine a number of inactive sampling intervals within several consecutive sampling periods, and determine that an physical performance activity period has ended (*e.g.,* the user is asleep) when a period of inactivity within each of the consecutive sampling periods exceeds a threshold time period. Each of the plurality of consecutive sampling periods includes a plurality of sampling intervals and processor 106 may be further configured to calculate a period of inactivity within each of the plurality of consecutive sampling periods based on motion data below a predetermined inactivity level.

To provide an illustrative example, if the activity goal period is a one-day period, the sampling periods are one-hour periods, and the sampling intervals are one-minute sampling intervals, processor 106 may execute instructions stored in motion analysis module 124 to determine that, over three consecutive hours, each of the one-hour sampling periods included a period of inactivity (by adding each sampling interval categorized as inactive) that exceeded a threshold time period T (*e.g.,* 25 minutes, 30 minutes, 35 minutes, etc.).

In other embodiments, processor 106 may execute instructions stored in motion analysis module 124 to calculate the reset time by additionally considering the active intervals within each of the consecutive sampling periods. That is, in some embodiments, processor 106 may analyze the inactivity intervals to determine a reset time when the aggregate time period represented by the sum of inactivity intervals within each of the consecutive sampling periods exceeds a threshold time period, as previously discussed. In accordance with such embodiments, processor 106 may not take into consideration the remaining active time intervals within each of the consecutive sampling periods.

But in other embodiments, processor 106 may analyze the remaining activity intervals within each of the consecutive sampling periods such that the reset time is calculated only when the remaining active intervals are at or below some threshold category (*e.g.*, moderate or below).

For example, embodiments include processor 106 not calculating a reset time if any of the remaining activity intervals within each of the consecutive sampling periods are categorized as high activity sampling intervals. These embodiments allow for flexibility in the determination of a reset time when a user is sleeping but gets up briefly in the middle of the night (reset time calculated) versus a user who may be napping before jogging (reset time not calculated).

Sensor analysis module 126 is a region of memory 118 configured to store instructions, that when executed by processor 106, cause processor 106 to perform various acts in accordance with applicable embodiments as described herein. In an embodiment, sensor analysis module 126 includes instructions that, when executed by processor 106, cause processor 106 to analyze sensor data generated by sensor array 108 over various sampling intervals. Embodiments include processor 106 determining a reset time based upon the sampled sensor data, which is further discussed below with reference to the instructions stored in reset time calculation module 128.

That is, in some embodiments, fitness monitoring device 102 may calculate a reset time based upon an analysis of motion data, as previously discussed. But in other embodiments, fitness monitoring device 102 may calculate a reset time based upon other sensor data generated by sensor array 108 in addition to or as an alternative to the analysis of motion data.

For example, embodiments include sensor array 108 being implemented with any suitable light intensity sensor configured to measure light intensity and to generate light intensity data. In an embodiment, the light intensity data may represent one or more metrics that indicate a light intensity detected by fitness monitoring device 102.

In some embodiments, when the light intensity measured by fitness monitoring device 102 is below a particular threshold for a number of consecutive sampling intervals (e.g., 30 minutes, an hour, etc.) within a single sampling period, then processor 106 may calculate a reset time. In other embodiments, processor 106 may calculate a reset time based upon the light intensity detected by fitness monitoring device 102 being below a particular intensity threshold for a threshold time period over a number of consecutive sampling periods (each including a number of sampling intervals), similar to the analysis of motion data, as previously discussed.

Reset time calculation module 128 is a region of memory 118 configured to store instructions that, when executed by processor 106, cause processor 106 to perform various acts in accordance with applicable embodiments as described herein. In an embodiment, reset time calculation module 128 includes instructions that, when executed by processor 106, cause processor 106 to calculate, maintain, adjust, and/or update the reset time.

In an embodiment, once a reset time is calculated, reset time calculation module 128 may include instructions that, when executed by processor 106, facilitate maintaining the initial reset time in accordance with any suitable recurring schedule to accommodate the fitness activity goal. To do so, embodiments include reset time calculation module 128 including instructions to facilitate the implementation of one or more software timers and/or software real-time clocks. Additionally or alternatively, processor 106 may utilize one or more hardware timers and/or hardware real-time clocks implemented by analog driver mechanics 112, integrated as part of processor 106, utilized by fitness monitoring device 102 but not shown in FIG. 1 for purposes of brevity, etc.

As previously discussed, fitness monitoring device 102 may automatically calculate a reset time based upon various types of sensor data. Embodiments include reset time calculation module 128 including instructions that facilitate the calculation of a specific initial reset time in various manners.

For example, when processor 106 determines that each number of consecutive sampling periods include a period of inactivity exceeding a threshold time period, processor 106 may execute instructions stored in reset time calculation module 128 to calculate the reset time as the start of the consecutive sampling periods, the end of the consecutive sampling periods, or any time associated with the highest level of inactivity (i.e., the least amount of movement). To provide an illustrative example, if the consecutive sampling periods span a time from midnight to 3:00 A.M. and the high level of inactivity occurs at 1:28 A.M., then processor 106 may calculate the reset time as either midnight, 3:00 A.M. or 1:28 AM.

In other embodiments, when processor 106 determines that the light intensity detected by fitness monitoring device 102 is below a particular threshold for a number of consecutive sampling intervals, then processor 106 may execute instructions stored in reset time calculation module 128 to calculate the reset time as the start of the consecutive sampling intervals or the end of the consecutive sampling intervals. To provide an illustrative example, if the consecutive sampling intervals span a time from midnight to 12:30 A.M., then processor 106 may calculate the reset time as either midnight or 12:30 A.M.

In still other embodiments, when processor 106 determines that the light intensity detected by fitness monitoring device 102 is below a particular intensity threshold for a threshold time period over a number of consecutive sampling periods (each including a number of sampling intervals), then processor 106 may execute instructions stored in reset time calculation module 128 to calculate the reset time as the start of the consecutive sampling periods or the end of the consecutive sampling periods. To provide an illustrative example, if the consecutive sampling periods span a time from midnight to 3:00 A.M., then processor 106 may calculate the reset time as either midnight or 3:00 A.M.

In yet additional embodiments, processor 106 may utilize additional data generated via sensor array 108 to modify the calculation of the reset time. For example, if sensor array 108 includes a barometer configured to generate pressure data, then processor 106 may sample this data similar to the sampling of motion data and light intensity data. Based upon this sampled data, processor 106 may calculate changes in elevation and determine that a user wearing fitness monitoring device 102 is flying on an airplane. Because long flights are known to disrupt sleeping patterns, embodiments include processor 106, upon making such a determination, tolling the sampling of motion data until some time period has passed after the user is back on the ground, not including a reset time calculated during a flight in an average reset time calculation, etc.

In some embodiments, once the reset time is calculated, processor 106 may store the calculated reset time as a reference in memory 118 and continue to track this time via a timer, a real-time clock, etc., to reset the tracking period associated with the fitness activity goal at this same reset time at the end of each activity goal period.

But in other embodiments, processor 106 may adjust the reset time over the course of several days or a plurality of fitness activity goal periods. For example, processor 106 may calculate an initial reset time using any of the techniques described herein for a first partial day or a first activity goal period. Processor 106 may continue to utilize the motion data over an extended period of time (e.g., 2 days, 4 days, 1 week, etc.) to calculate additional reset times (again, using any techniques described herein) to calculate an approximate midnight time and adjust the initial reset time based upon these subsequent calculations to equal an approximated midnight time.

In an embodiment, processor 106 may perform an initial calibration period in which a number of reset times are averaged, updating the initial reset time with the averaged reset time at the end of the calibration period. To provide another example, processor 106 may continuously calculate reset times and update the reset time each subsequent activity goal period using a rolling average of previously calculated reset times stored in memory 118. In this way, processor 106 may "fine tune" the reset time over time as additional data is analyzed, thereby continually improving its accuracy. In embodiments, the calculated reset time may be refined after a predetermined length of time (e.g., approximately 24 hours) by processor 106 returning to the step of analyzing new motion data over a sampling interval.

Regardless of how the reset time is maintained, embodiments include the activity goal reset time being maintained independently of the time-of-day that may otherwise be maintained by fitness monitoring device 102.

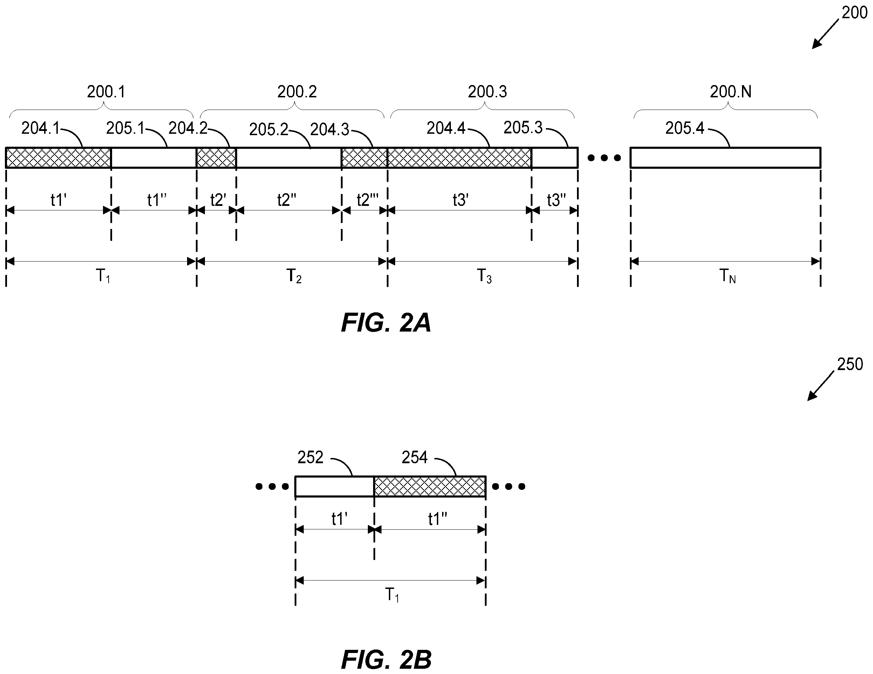

FIG. 2A is an illustration of exemplary consecutive data sampling periods 200 for calculating a reset time, according to an embodiment. In an embodiment, consecutive data sampling periods 200 include any suitable number $N$ of sampling periods 200.1-200.N, which may each represent a respective sampling time $T_1$ - $T_N$ of an hour, a half hour, etc. Further in accordance with such an embodiment, each of data sampling periods 200.1-200.N may include any suitable number of sampling intervals, which may represent sampling intervals, for example, such as one minute sampling intervals, 30-second sampling intervals, etc.

In an embodiment, the sampling intervals within each sampling period 200 may correspond to time periods in which motion data is sampled and analyzed by processor 106. For example, each sampling period 200 may include sampling intervals that have been categorized as active intervals or as inactive intervals based upon an analysis of motion data occurring over each respective sampling interval.

As shown in FIG. 2A, sampling period 200.1, which occurs over a sampling time $T_1$, includes sampling intervals 204.1 that have been categorized as inactive intervals and occur over a time period t1' within sampling period 200.1. Sampling period 200.1 also includes sampling intervals 205.1 that have been categorized as active intervals and occur over a time period t1" within sampling period 200.1. Thus, the time represented by the aggregation of time corresponding to both active intervals and inactive intervals within each sampling period represents the total time of the sampling period, which is $T_1$ in this example.

Continuing the example shown in FIG. 2A, sampling period 200.2, which occurs over a sampling time $T_2$, includes sampling intervals 204.2 and 204.3, which have been categorized as inactive intervals and which occur over a time period t2' + t2''' within sampling period 200.2. Sampling period 200.2 also includes sampling intervals 205.2 that have been categorized as active intervals and occur over a time period t2" within sampling period 200.2.

Furthermore, sampling period 202.3, which occurs over a sampling time $T_3$, includes sampling intervals 204.4 that have been categorized as inactive intervals and occur over a time period t3' within sampling period 200.3. Sampling period 200.3 also includes sampling intervals 205.3 that have categorized as active intervals and occur over a time period t3" within sampling period 200.3.

Using the exemplary sampling times $T_1$ - $T_N$ and sampling interval time periods t shown in FIG. 2A, embodiments include processor 106 calculating a reset time when each of the time periods represented by the aggregated inactive intervals 204.1-204.4, within each of their respective sampling periods 200.1-200.3, exceed a threshold time period.

For example, each of the consecutive sampling periods 200.1-200.3 may represent a one-hour time period, while the sampling intervals within each of the sampling periods represent one-minute sampling intervals. In an embodiment, a threshold inactivity time period I may be specified (e.g., as part of the instructions stored in reset time calculation module 128) and processor 106 may calculate a reset time when each of the following is true:

$$t1' > I;$$

$$(t2' + t2''') > I;$$

and

$$t3' > I.$$

Again, in various embodiments, the threshold time period I may be any suitable time period, such as 30 minutes, 35 minutes, etc. Although sampling period 200.2 includes two time periods of inactivity (t2" and t2''')

that are separated by a time period of activity (t2"), embodiments include processor 106 calculating the reset time as long as the sum of t2" and t2‴ is greater than I. In this way, embodiments allow for some flexibility in the determination of the reset time, allowing for trips during the middle of the night, restless sleepers wearing the fitness monitoring device while sleeping, etc.

Once processor 106 determines that the above conditions have been met, embodiments include processor 106 calculating the reset time using any suitable relationship between sampling periods 200.1-200.3, such as the start of sampling period 200.1, the end of sampling period 200.3, etc.

FIG. 2B is an illustration of exemplary single data sampling period 250 for calculating a reset time, according to an embodiment. In an embodiment, data sampling period 250 may represent a sampling time $T_1$ of an hour, a half hour, etc. Further in accordance with such an embodiment, data sampling period 250 may include any suitable number of sampling intervals, which may include, for example, one minute sampling intervals, 30-second sampling intervals, etc.

In an embodiment, the sampling intervals included in data sampling period 250 may include light intensity data that is sampled and analyzed by processor 106. For example, sampling period 250 may include sampling intervals that have been categorized as having a measured light intensity below or above a threshold light intensity. As shown in FIG. 2B, sampling period 250 includes a grouping of sampling intervals 252 that have been categorized by processor 106 as being above a threshold light intensity over a time period within sampling period 250 of t1'. Sampling period 250 includes sampling intervals 254 that have been categorized by processor 106 as being below a threshold light intensity over a sampling time period within sampling period 250 of t1".

Using the exemplary data sampling period 250 as shown in FIG. 2B, embodiments include processor 106 calculating a reset time when the time period associated with sampling intervals 254 add up to a time period that exceeds a threshold time period I. As previously discussed with reference to FIG. 2A, the threshold time period I may be any suitable time period, such as 30 minutes, 35 minutes, etc.

Once processor 106 determines that the time period associated with sampling intervals 254 exceed the threshold time period, embodiments include processor 106 calculating the reset time using any suitable time based upon sampling period 250, such as the start of time period t1", the end of sampling period 250, the end of time period t1", etc.

Although a single data sampling period 250 is shown in FIG. 2A, embodiments include processor 106 analyzing the light intensity data over a plurality of sampling periods. In accordance with such embodiments, processor 106 may calculate a reset time based upon the sampling intervals, categorized as below a threshold light intensity, exceeding a threshold time period over several consecutive sampling periods, as discussed above with regards to the analysis of motion data in FIG. 2A.

FIG. 3 is an illustration of an exemplary fitness monitoring device 300 in accordance with an embodiment of the present disclosure. In an embodiment, exemplary fitness monitoring device 300 is an implementation of fitness monitoring device 102, as shown in FIG. 1.

Fitness monitoring device 300 includes a left arc-shaped window 302, which displays an indication of a user's activity, and a right arc-shaped window 304, which displays an indication of a user's inactivity.

Left arc-shaped window 302 may include portions 302A and 302B, which display various colors, textures, patterns, labels, indicia, etc., to indicate the user's activity based upon the position of portions 302A and 302B. For example, portion 302A may represent a color corresponding to a fitness activity goal not being met, while portion 302B may represent a color corresponding the fitness activity goal being met. Continuing this example, fitness monitoring device 300 may be configured to display portion 302B in its entirety within left arc-shaped window 302 once the motion data indicates that the user has reached the threshold number of steps associated with the fitness activity goal, while displaying portion 302A in its entirety within left arc-shaped window 302 when the motion data indicates that the user has taken no steps so far towards accomplishing the fitness activity goal.

The right arc-shaped window 304 may include portions 304A and 304B, which display various colors, textures, patterns, labels, indicia, etc., to indicate the user's inactivity based upon the position of portions 304A and 304B. For example, portion 304A may represent no accrual of inactivity time, while portion 304A may represent a time in which a user has remained inactive. Continuing this example, fitness monitoring device 300 may be configured to display portion 304A in its entirety within right arc-shaped window 304 until a user has been inactive for some threshold amount of time, in which case a greater amount of portion 304B will continue to be visible until a user begins taking steps again.

Again, fitness monitoring device 300 may be configured to track steps and/or inactivity time throughout a period of time in accordance with a fitness activity goal schedule, such as over a one-day period, for example. This tracking may be performed over the fitness activity goal schedule starting at the calculated reset time, and repeating periodically in accordance with the fitness activity goal schedule (e.g., each day). Therefore, embodiments include fitness monitoring device 300 resetting the number of steps to zero and the inactivity time to zero, etc., upon passage of the calculated reset time, such that only portions 302A and 304A are shown through left arc shaped window 302 and right arc shaped window 304, respectively.

The portions 302A and 302B displayed in left arc-shaped window 302, as well as the portions 304A and 304B displayed in right arc-shaped window 304, may be controlled via any suitable mechanical and/or electromechanical systems, such as any suitable combination of rotating discs, couplings, gears, motors, etc. The various suitable mechanical and/or electromechanical structures implemented by fitness monitoring device 300 may be represented, for example, by analog drive mechanics 112, as shown in FIG. 1, which may also facilitate the movement of the watch hands.

FIG. 4 is an illustration of an exemplary fitness monitoring device 400 in accordance with an embodiment of the present disclosure. In an embodiment, exemplary fitness monitoring device 400 is an implementation of fitness monitoring device 102, as shown in FIG. 1. In an embodiment, fitness monitoring device 400 may include a band 402, a protective bezel 404, and a display 406. In an embodiment, display 406 may be an implementation of display 110, as shown in FIG. 1.

Fitness monitoring device 400 may display information on display 406 such as, for example, a variable portion of display 406 (410) that may display various information based upon a user selection, such as physical performance information. For instance, display 406 ay present a number of steps remaining in an activity goal period and/or a number of steps taken. Display 406 may also include an indication of whether steps are being counted based on the motion data (412), an indication of what is being displayed in the selectable portion of display 406 (414), a graphical representation of the user's progress towards the activity goal (416), etc.

In an embodiment, fitness monitoring device 400 may display similar or identical information as fitness monitoring device 300, but use a digital format as opposed to the analog format of fitness monitoring device 300. Again, fitness monitoring device 400 may be configured to track steps and/or inactivity time throughout a period of time in accordance with a fitness activity goal schedule, such as over a 24-hour period, for example. This tracking may be performed over the fitness activity goal schedule starting at the calculated reset time, repeating periodically in accordance with the fitness activity goal schedule. Therefore, embodiments include fitness monitoring device 400 resetting to zero the physical performance information, such as a number of calculated steps, the user's progress towards the activity goal (416), and the number of steps remaining in an activity goal period upon passage of the calculated reset time.

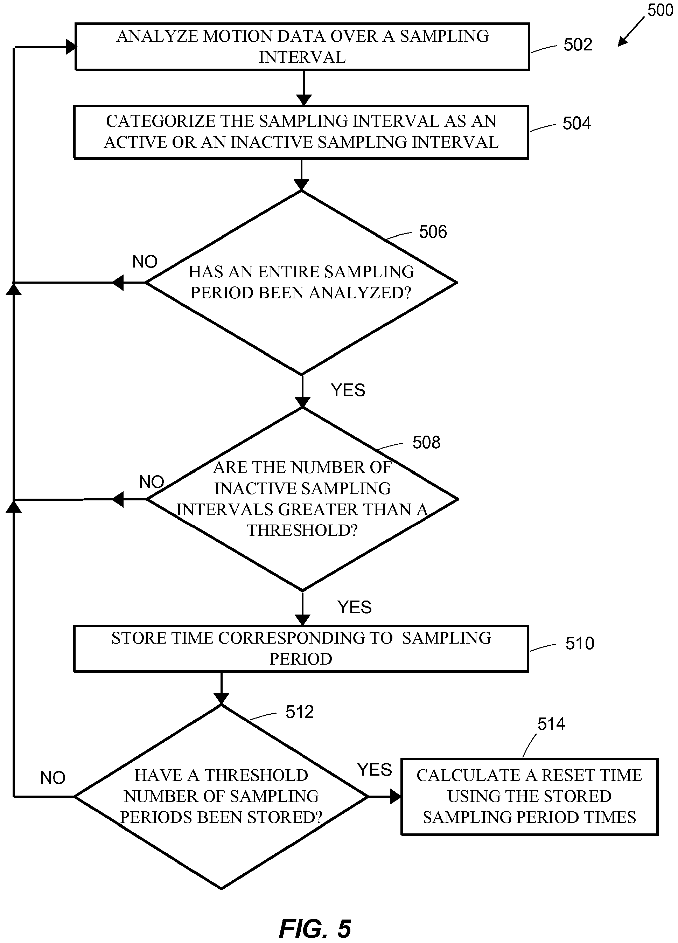

FIG. 5 illustrates a method flow 500, according to an embodiment. In an embodiment, one or more regions of method 500 (or the entire method 500) may be implemented by any suitable device. For example, one or more regions of method 500 may be performed by fitness monitoring device 102, as shown in FIG. 1.

Method 500 may start when one or more processors analyze motion data over a sampling interval (block 502). This motion data may include, for example, data received from a one or more sensors (e.g., accelerometers), such as sensor array 108, for example, as shown in FIG. 1 (block 502).

Method 500 may include one or more processors categorizing the sampling interval as an active or an inactive sampling interval (block 504). This may include, for example, categorizing a sampling interval of physical performance information as an inactive interval when no steps are calculated over the entire sampling interval based upon the motion data analyzed during this time (block 504). This may also include, for example, categorizing a sampling interval of physical performance information as an active interval when the number of steps calculated over the entire sampling interval based upon the motion data analyzed during this time are greater than zero (block 504).

Method 500 may include one or more processors determining whether an entire sampling period has been analyzed (block 506). This may include, for example, one or more processors maintaining a count of the number of categorized sampling intervals (blocks 502 and 504) and comparing this count to a number associated with the number of sampling intervals within a sampling period (block 506).

For example, if the sampling period is one hour, then method 500 may include one or more processors determining that the entire sampling period has been analyzed when 60 sampling intervals have been categorized (block 506).

If so, then method 500 may continue to determine whether the number of inactive sampling intervals within the sampling period exceed a threshold number (block 508). Otherwise, method 500 may revert to continuing to analyze motion data over the next sampling interval (block 502).

Method 500 may include one or more processors determining whether the number of inactive sampling intervals within the previous sampling period exceed a threshold number (block 508). Again, using the example of a one hour sampling period, method 500 may include one or more processors determining that the number of inactive sampling intervals exceeds a threshold number of 30 minutes, 35 minutes, etc. (block 508).

If so, then method 500 may continue to store a time corresponding to the sampling period (block 510). Otherwise, method 500 may revert to continuing to analyze motion data over the next sampling interval (block 502), which is now with regards to the next sampling period.

Method 500 may include one or more processors storing a time corresponding to the sampling period (block 510). This time may be stored in any suitable format, such as a reference to a time, a real-time clock, timestamped data, etc. (block 510).

The time may be stored in any suitable storage device, such as memory 118, for example, as shown in FIG. 1. Embodiments include the time stored being independent and separate from other clocks utilized by the fitness monitoring device in which method 500 is implemented (block 510).

Method 500 may include one or more processors determining whether a threshold number of sampling periods have been stored (block 512). This may include, for example, comparing the time stamps to data indicative of the sampling period times.

Based on this comparison, a determination may be made whether a threshold number (e.g., 2, 3, 4, etc.) of concurrent sampling periods have passed (block 510), each of which including a number of inactive sampling intervals greater than a (separate) threshold (block 508).

If so, then method 500 may continue to calculate a reset time (block 514). Otherwise, method 500 may revert to continuing to analyze motion data over the next sampling interval (block 502), which is now with regards to the next sampling period.

Method 500 may include one or more processors calculating a reset time (block 514) based upon the stored number of sampling periods exceeding a threshold (block 512). This may include, for example, calculating a reset time using the beginning of the concurrent sampling periods, using the end of the concurrent sampling periods, etc., as previously discussed with reference to FIG. 2 (block 514).

In embodiments, the calculated reset time (block 514) may be refined after a predetermined length of time (e.g., approximately 24 hours) when the method 500 returns to the initial step of one or more processors analyze new motion data over a sampling interval (block 502).

Although the foregoing text sets forth a detailed description of numerous different embodiments, it should be understood that the detailed description is to be construed as exemplary only and does not describe every possible embodiment because describing every possible embodiment would be impractical, if not impossible.

In light of the foregoing text, numerous alternative embodiments may be implemented, using either current technology or technology developed after the filing date of this patent application.

**Claims**

1.   A fitness-monitoring device (102, 300, 400), comprising:

a sensor (108) configured to measure movements of a user wearing the fitness monitoring device (102, 300, 400) and to generate motion data;
a memory (118) configured to store the motion data and physical performance information; and
a processor (106) configured to:

determine the physical performance information based on the motion data;

track the physical performance information over a predetermined period of time;
analyze the motion data over a plurality of sampling intervals and a plurality of consecutive sampling periods, wherein each of the sampling periods is made up of multiple of the sampling intervals such that the respective multiple sampling intervals constitute the respective sampling period from among the plurality of consecutive sampling periods;
categorize each of the plurality of sampling intervals as an active sampling interval or an inactive sampling interval based upon the motion data analyzed over each respective sampling interval;
calculate, utilizing the motion data, a reset time when, within each of a plurality of consecutive sampling periods, the number of inactive sampling intervals exceeds a threshold number; and
reset the tracked physical performance information to zero at the reset time to restart tracking of the physical performance information for an approximate one-day period.

2.   The fitness-monitoring device of claim 1, wherein the processor (106) is further configured to determine, utilizing the motion data stored over an extended period of time, an approximated midnight time and adjust the reset time to equal the approximated midnight time, wherein preferably the sampling period is one hour, the predetermined period of time is less than twenty-four hours and the extended period of time is four days.

3.   The fitness-monitoring device of claim 1, wherein the processor (106) is configured to calculate the reset time as the start of the first of the plurality of consecutive sampling periods in which the number of inactive sampling intervals exceeds the threshold number.

4.   The fitness-monitoring device of claim 1, wherein the processor (106) is further configured to calculate an average reset time using a moving average of reset times calculated from a plurality of periods of time and to update the reset time with the average reset time, and/or
wherein the fitness-monitoring device (102, 300, 400) maintains and displays a time of day, and the processor (106) is further configured to calculate the reset time independent of the time of day.

5.   The fitness-monitoring device of claim 1, wherein each of the plurality of consecutive sampling periods includes a plurality of one minute sampling intervals, and

wherein the processor (106) is further configured to calculate a period of inactivity within each of the plurality of consecutive sampling periods based on motion data below a predetermined inactivity level.

6. The fitness-monitoring device of claim 5, wherein each of the plurality of consecutive sampling periods constitutes a respective period of inactivity and a respective period of activity, and
   wherein the processor (106) is further configured to calculate the period of activity within each of the plurality of consecutive sampling periods as an aggregation of time corresponding to sampling intervals over which the motion data indicates that steps are taken by the user.

7. The fitness-monitoring device of claim 6, wherein:

   the processor (106) is further configured to classify the period of activity within each of the plurality of consecutive sampling periods as a period of moderate activity or a period of strenuous activity,
   the period of moderate activity corresponding to an aggregation of time intervals over which the motion data exceed a predetermined inactivity level,
   the period of strenuous activity corresponding to an aggregation of time intervals over which the motion data exceed a predetermined inactivity level and a strenuous activity threshold, and
   the processor (106) is further configured to calculate the reset time when each of the plurality of consecutive sampling periods includes no period of strenuous activity.

8. A fitness-monitoring device, comprising:

   a sensor array (108) configured to:

   measure movements of a user wearing the fitness monitoring device (102, 300, 400) and to generate motion data; and
   measure light intensity and to generate light intensity data;

   a memory (118) configured to store light intensity data and physical performance information; and
   a processor (106) configured to:

   determine the physical performance information based on the motion data;
   track physical performance information over a predetermined period of time;
   analyze the light intensity data over a plurality of sampling intervals and a plurality of consecutive sampling periods, wherein each of the sampling periods is made up of multiple of the sampling intervals such that the respective multiple sampling intervals constitute the respective sampling period from among the plurality of consecutive sampling periods;
categorize each of the plurality of sampling intervals based upon the light intensity data analyzed over each respective sampling interval as a sampling interval having a measured light intensity below a light intensity threshold or a sampling interval having a measured light intensity above the light intensity threshold;
calculate, utilizing the light intensity data, a reset time when, within each of a plurality of consecutive sampling periods, the number of sampling intervals having the measured light intensity below the light intensity threshold exceeds a threshold number; and
reset the tracked physical performance information to zero at the reset time to restart tracking the physical performance information for an approximate one-day period.

9. The fitness-monitoring device of claim 8, wherein the processor (106) is further configured to determine, utilizing the light intensity data stored over an extended period of time, an approximated midnight time and adjust the reset time to equal the approximated midnight time, and/or
   wherein the processor (106) is configured to calculate the reset time as the start of the first of the plurality of consecutive sampling periods in which the number of inactive sampling intervals exceeds the threshold number.

10. The fitness-monitoring device of claim 1 or claim 8, wherein the physical performance information is the number of steps taken by the user, the number of steps being part of a fitness activity goal that represents a threshold number of steps to be taken by the user over the one-day period.

11. The fitness-monitoring device of claim 11, wherein the sampling period is one hour.

12. The fitness-monitoring device of claim 8, wherein the processor (106) is configured to calculate an average reset time using a moving average of reset times calculated from a plurality of periods of time and to update the reset time with the average reset time.

13. A computer-implemented method in a fitness-monitoring device (102, 300, 400), comprising:

generating, using a sensor (108) in the fitness-monitoring device (102, 300, 400), motion data based upon measured movements of a user wearing the fitness-monitoring device (102, 300, 400);

determining, using a processor (106) in the fitness-monitoring device (102, 300, 400), physical performance information based on the motion data;

tracking, using the processor (106), physical performance information over a predetermined period of time;

analyzing, using the processor (106), the motion data over a plurality of sampling intervals and a plurality of consecutive sampling periods, wherein each of the sampling periods is made up of multiple of the sampling intervals such that the respective multiple sampling intervals constitute the respective sampling period from among the plurality of consecutive sampling periods;

categorizing, using the processor (106), each of the plurality of sampling intervals as an active sampling interval or an inactive sampling interval based upon the motion data analyzed over each respective sampling interval;

calculating, using the processor (106), a reset time when, within each of the plurality of consecutive sampling periods, the number of inactive sampling intervals exceeds a threshold number; and

resetting, using the processor (106), the tracked physical performance information to zero at the reset time to restart tracking the physical performance information for an approximate one-day period.

14. The computer-implemented method of claim 13, further comprising determining, utilizing the motion data stored over an extended period of time, an approximated midnight time and adjusting the reset time to equal the approximated midnight time.

15. The computer-implemented method of claim 13, wherein physical performance information is the number of steps taken by the user, the number of steps being part of a fitness activity goal that represents a threshold number of steps to be taken by the user over a one day period; and wherein each of the plurality of sampling periods is one hour, each of the plurality of sampling intervals is one minute and the extended period of time is four days, wherein preferably categorizing each of the plurality of sampling intervals comprises the processor (106):

categorizing a sampling interval as an inactive interval when the motion data analyzed over the sampling interval indicates that no steps were taken by the user; and

categorizing a sampling interval as an active interval when the motion data analyzed over the sampling interval indicates that steps were taken by the user.

**Patentansprüche**

1. Fitnessüberwachungsvorrichtung (102, 300, 400), die Folgendes umfasst:

einen Sensor (108), der so konfiguriert ist, dass er Bewegungen eines Benutzers, der die Fitnessüberwachungsvorrichtung (102, 300, 400) trägt, misst und Bewegungsdaten erzeugt;

einen Speicher (118), der so konfiguriert ist, dass er die Bewegungsdaten und physische Leistungsinformationen speichert; und

einen Prozessor (106), der so konfiguriert ist, dass er:

die physischen Leistungsinformationen auf der Basis der Bewegungsdaten bestimmt;

die physischen Leistungsinformationen über einen vorbestimmten Zeitraum verfolgt;

die Bewegungsdaten über eine Mehrzahl von Abtastintervallen und eine Mehrzahl von aufeinanderfolgenden Abtastperioden analysiert, wobei jede der Abtastperioden mit einem Mehrfachen der Abtastintervalle gebildet ist, so dass die jeweiligen mehrfachen Abtastintervalle die jeweilige Abtastperiode aus der Mehrzahl der aufeinanderfolgenden Abtastperioden bilden;

jedes der mehreren Abtastintervalle als aktives Abtastintervall oder als inaktives Abtastintervall auf der Basis der über das jeweilige Abtastintervall analysierten Bewegungsdaten kategorisiert;

unter Verwendung der Bewegungsdaten eine Rücksetzzeit berechnet, wenn innerhalb jeder einer Mehrzahl von aufeinanderfolgenden Abtastperioden die Anzahl der inaktiven Abtastintervalle eine Schwellenzahl überschreitet; und

die verfolgten physischen Leistungsinformationen zur Rücksetzzeit auf Null zurücksetzt, um die Verfolgung der physischen Leistungsinformationen für einen Zeitraum von etwa einem Tag neu zu starten.

2. Fitnessüberwachungsvorrichtung nach Anspruch 1, wobei der Prozessor (106) ferner so konfiguriert ist, dass er unter Verwendung der über einen längeren Zeitraum gespeicherten Bewegungsdaten eine ungefähre Mitternachtszeit bestimmt und die Rücksetzzeit so einstellt, dass sie der ungefähren Mitternachtszeit entspricht, wobei die Abtastperiode vorzugsweise eine Stunde beträgt, der vorbestimmte Zeitraum weniger als vierundzwanzig Stunden beträgt und der längere Zeitraum vier Tage beträgt.

3. Fitnessüberwachungsvorrichtung nach Anspruch 1, wobei der Prozessor (106) so konfiguriert ist, dass er die Rücksetzzeit als den Beginn der ersten von mehreren aufeinanderfolgenden Abtastperioden berechnet, in denen die Anzahl der inaktiven Abtastintervalle die Schwellenzahl überschreitet.

4. Fitnessüberwachungsvorrichtung nach Anspruch 1, wobei der Prozessor (106) ferner so konfiguriert ist, dass er eine durchschnittliche Rücksetzzeit unter Verwendung eines gleitenden Durchschnitts von Rücksetzzeiten, die aus einer Mehrzahl von Zeiträumen berechnet wurden, berechnet und die Rücksetzzeit mit der durchschnittlichen Rücksetzzeit aktualisiert, und/oder wobei die Fitnessüberwachungsvorrichtung (102, 300, 400) eine Tageszeit beibehält und anzeigt, und der Prozessor (106) ferner so konfiguriert ist, dass er die Rücksetzzeit unabhängig von der Tageszeit berechnet.

5. Fitnessüberwachungsvorrichtung nach Anspruch 1, wobei jede der mehreren aufeinanderfolgenden Abtastperioden mehrere einminütige Abtastintervalle umfasst, und wobei der Prozessor (106) ferner so konfiguriert ist, dass er eine Inaktivitätsperiode innerhalb jeder der mehreren aufeinanderfolgenden Abtastperioden auf der Grundlage von Bewegungsdaten unterhalb eines vorbestimmten Inaktivitätsniveaus berechnet.

6. Fitnessüberwachungsvorrichtung nach Anspruch 5, wobei jede der mehreren aufeinanderfolgenden Abtastperioden eine jeweilige Inaktivitätsperiode und eine jeweilige Aktivitätsperiode darstellt, und wobei der Prozessor (106) ferner so konfiguriert ist, dass er die Aktivitätsperiode innerhalb jeder der mehreren aufeinanderfolgenden Abtastperioden als eine Ansammlung von Zeit berechnet, die Abtastintervallen entspricht, über die die Bewegungsdaten anzeigen, dass Schritte vom Benutzer unternommen werden.

7. Fitnessüberwachungsvorrichtung nach Anspruch 6, wobei:

der Prozessor (106) ferner so konfiguriert ist, dass er die Aktivitätsperiode innerhalb jeder der mehreren aufeinanderfolgenden Abtastperioden als eine Periode mäßiger Aktivität oder als eine Periode anstrengender Aktivität klassifiziert,

die Periode mäßiger Aktivität entspricht einer Ansammlung von Zeitintervallen, in denen die Bewegungsdaten ein vorbestimmtes Inaktivitätsniveau überschreiten, die Periode anstrengender Aktivität, die einer Ansammlung von Zeitintervallen entspricht, in denen die Bewegungsdaten ein vorbestimmtes Inaktivitätsniveau und einen Schwellenwert für anstrengende Aktivität überschreiten, und der Prozessor (106) ferner so konfiguriert ist, dass er die Rücksetzzeit berechnet, wenn jede der mehreren aufeinanderfolgenden Abtastperioden keine Periode anstrengender Aktivität enthält.

8. Fitnessüberwachungsvorrichtung, die Folgendes umfasst:

einen Sensor (108), der so konfiguriert ist, dass er:

Bewegungen eines Benutzers, der die Fitnessüberwachungsvorrichtung (102, 300, 400) trägt, misst und Bewegungsdaten erzeugt; und Lichtintensität misst und Lichtintensitätsdaten erzeugt;

einen Speicher (118), der so konfiguriert ist, dass er Lichtintensitätsdaten und physische Leistungsinformationen speichert; und einen Prozessor (106), der so konfiguriert ist, dass er:

die physischen Leistungsinformationen auf der Grundlage der Bewegungsdaten bestimmt; die physische Leistungsinformationen über einen bestimmten Zeitraum hinweg verfolgt; die Lichtintensitätsdaten über eine Mehrzahl von Abtastintervallen und eine Mehrzahl von aufeinanderfolgenden Abtastperioden analysiert, wobei jede der Abtastperioden mit einem Mehrfachen der Abtastintervalle gebildet ist, so dass die jeweiligen mehrfachen Abtastintervalle die jeweilige Abtastperiode aus der Mehrzahl der aufeinanderfolgenden Abtastperioden bilden; jedes der mehreren Abtastintervalle auf Basis der über jedes jeweilige Abtastintervall analysierten Lichtintensitätsdaten als Abtastintervall mit einer gemessenen Lichtintensität unterhalb eines Lichtintensitätsschwellenwerts oder als Abtastintervall mit einer gemessenen Lichtintensität oberhalb des Lichtintensitätsschwellenwerts kategorisiert;

unter Verwendung der Lichtintensitätsdaten eine Rücksetzeit berechnet, wenn innerhalb jeder einer Mehrzahl von aufeinanderfolgenden Abtastperioden die Anzahl von Abtastintervallen, in denen die gemessene Lichtintensität unter dem Lichtintensitätsschwellenwert liegt, eine Schwellenzahl überschreitet; und

die verfolgten physischen Leistungsdaten zur Rücksetzzeit auf Null zurücksetzt, um die Verfolgung der physischen Leistungsinformationen für einen Zeitraum von etwa einem Tag neu zu starten.

9. Fitnessüberwachungsvorrichtung nach Anspruch 8, wobei der Prozessor (106) ferner so konfiguriert ist, dass er unter Verwendung der über einen längeren Zeitraum gespeicherten Lichtintensitätsdaten eine ungefähre Mitternachtszeit bestimmt und die Rücksetzzeit so einstellt, dass sie der ungefähren Mitternachtszeit entspricht, und/oder

wobei der Prozessor (106) so konfiguriert ist, dass er die Rücksetzzeit als den Beginn der ersten von mehreren aufeinanderfolgenden Abtastperioden berechnet, in denen die Anzahl der inaktiven Abtastintervalle die Schwellenzahl überschreitet.

10. Fitnessüberwachungsvorrichtung nach Anspruch 1 oder Anspruch 8, wobei die Information über die physische Leistungsinformationen die Anzahl der vom Benutzer gemachten Schritte ist, wobei die Anzahl der Schritte Teil eines Fitnessaktivitätsziels ist, das eine Schwellenanzahl von Schritten darstellt, die vom Benutzer über den Zeitraum eines Tages gemacht werden sollen.

11. Fitnessüberwachungsvorrichtung nach Anspruch wobei die Abtastperiode eine Stunde beträgt.

12. Fitnessüberwachungsvorrichtung nach Anspruch 8, wobei der Prozessor (106) so konfiguriert ist, dass er eine durchschnittliche Rücksetzzeit unter Verwendung eines gleitenden Durchschnitts von Rücksetzzeiten, die aus einer Mehrzahl von Zeiträumen berechnet wurden, berechnet und die Rücksetzzeit mit der durchschnittlichen Rücksetzzeit aktualisiert.

13. Computerimplementiertes Verfahren in einer Fitnessüberwachungsvorrichtung (102, 300, 400), umfassend:

Erzeugen, unter Verwendung eines Sensors (108) in der Fitnessüberwachungsvorrichtung (102, 300, 400), von Bewegungsdaten, die auf gemessenen Bewegungen eines Benutzers basieren, der die Fitnessüberwachungsvorrichtung (102, 300, 400) trägt;
Bestimmen, unter Verwendung eines Prozessors (106) in der Fitnessüberwachungsvorrichtung (102, 300, 400), von physischen Leistungsinformationen auf Basis der Bewegungsdaten;
Verfolgung, unter Verwendung des Prozessors (106), von physischen Leistungsinformationen über einen vorbestimmten Zeitraum;
Analysieren, unter Verwendung des Prozessors (106), der Bewegungsdaten über eine Mehrzahl von Abtastintervallen und eine Mehrzahl von aufeinanderfolgenden Abtastperioden, wobei jede der Abtastperioden aus einem Mehrfachen der Abtastintervalle besteht, so dass die jeweiligen mehrfachen Abtastintervalle die jeweilige Abtastperiode aus der Mehrzahl der aufeinanderfolgenden Abtastperioden bilden;
Kategorisieren, unter Verwendung des Prozessors (106), jedes der Mehrzahl von Abtastintervallen als ein aktives Abtastintervall oder ein inaktives Abtastintervall, basierend auf den Bewegungsdaten, die über jedes jeweilige Abtastintervall analysiert wurden;
Berechnen, unter Verwendung des Prozessors (106), einer Rücksetzzeit, wenn innerhalb jeder der mehreren aufeinanderfolgenden Abtastperioden die Anzahl der inaktiven Abtastintervalle eine Schwellenzahl überschreitet; und
Zurücksetzen, unter Verwendung des Prozessors (106), der verfolgten physischen Leistungsinformationen zum Rücksetzzeitpunkt auf Null, um die Verfolgung der physischen Leistungsinformationen für einen ungefähren Zeitraum von einem Tag neu zu starten.

14. Computerimplementiertes Verfahren nach Anspruch 13, das ferner die Bestimmung einer ungefähren Mitternachtszeit unter Verwendung der über einen längeren Zeitraum gespeicherten Bewegungsdaten und die Anpassung der Rücksetzzeit umfasst, um der ungefähren Mitternachtszeit zu entsprechen.

15. Computerimplementiertes Verfahren nach Anspruch 13, wobei es sich bei den physischen Leistungsinformationen um die Anzahl der vom Benutzer unternommenen Schritte handelt, wobei die Anzahl der Schritte Teil eines Fitnessaktivitätsziels ist, das einen Schwellenwert für die Anzahl der vom Benutzer über einen Zeitraum von einem Tag zu unternehmenden Schritte darstellt; und wobei jede der mehreren Abtastperioden eine Stunde beträgt, jedes der mehreren Abtastintervalle eine Minute beträgt und der erweiterte Zeitraum vier Tage beträgt, wobei vorzugsweise die Kategorisierung jedes der Vielzahl von Abtastintervallen den Prozessor (106) umfasst:

Kategorisieren eines Abtastintervalls als ein inaktives Intervall, wenn die über das Abtastintervall analysierten Bewegungsdaten anzeigen, dass keine Schritte durch den Benutzer unternommen wurden; und

Einstufung eines Abtastintervalls als aktives Intervall, wenn die während des Abtastintervalls analysierten Bewegungsdaten darauf hinweisen, dass der Benutzer Schritte unternommen hat.

## Revendications

1. Dispositif de surveillance d'aptitude (102, 300, 400), comprenant :

un capteur (108) configuré pour mesurer les mouvements d'un utilisateur portant le dispositif de surveillance d'aptitude physique (102, 300, 400) et pour générer des données de mouvement ;
une mémoire (118) configurée pour stocker les données de mouvement et les informations de performance physique ; et
un processeur (106) configuré pour :

déterminer les informations de performance physique sur la base des données de mouvement ;
suivre les informations de performance physique sur une période de temps prédéterminée ;
analyser les données de mouvement sur une pluralité d'intervalles d'échantillonnage et une pluralité de périodes d'échantillonnage consécutives, dans lesquelles chacune des périodes d'échantillonnage est composée d'un multiple des intervalles d'échantillonnage de sorte que les intervalles d'échantillonnage multiples respectifs constituent la période d'échantillonnage respective parmi la pluralité de périodes d'échantillonnage consécutives ;
catégoriser chacun de la pluralité d'intervalles d'échantillonnage comme un intervalle d'échantillonnage actif ou un intervalle d'échantillonnage inactif sur la base des données de mouvement analysées sur chaque intervalle d'échantillonnage respectif ;
calculer, à l'aide des données de mouvement, un temps de réinitialisation lorsque, au cours de chacune d'une pluralité de périodes d'échantillonnage consécutives, le nombre d'intervalles d'échantillonnage inactifs dépasse un nombre seuil ; et
réinitialiser les informations de performance physique suivies à zéro au moment de la réinitialisation pour faire redémarrer le suivi des informations de performance physique pendant une période approximative d'une journée.

2. Dispositif de surveillance d'aptitude selon la revendication 1, dans lequel le processeur (106) est configuré en outre pour déterminer, en utilisant les données de mouvement stockées sur une période de temps étendue, un temps de minuit approximatif et ajuster le temps de réinitialisation pour qu'il soit égal au temps de minuit approximatif, dans lequel, de préférence, la période d'échantillonnage est d'une heure, la période de temps prédéterminée est inférieure à vingt-quatre heures et la période de temps étendue est de quatre jours.

3. Dispositif de surveillance d'aptitude selon la revendication 1, dans lequel le processeur (106) est configuré pour calculer le temps de réinitialisation en tant que début de la première de la pluralité de périodes d'échantillonnage consécutives dans lesquelles le nombre d'intervalles d'échantillonnage inactifs dépasse le nombre de seuil.

4. Dispositif de surveillance d'aptitude selon la revendication 1, dans lequel le processeur (106) est configuré en outre pour calculer un temps de réinitialisation moyen en utilisant une moyenne mobile de temps de réinitialisation calculée à partir d'une pluralité de périodes de temps et pour mettre à jour le temps de réinitialisation avec le temps de réinitialisation moyen, et/ou dans lequel le dispositif de surveillance d'aptitude physique (102, 300, 400) maintient et affiche une heure de la journée, et le processeur (106) est configuré en outre pour calculer l'heure de réinitialisation indépendamment de l'heure de la journée.

5. Dispositif de surveillance d'aptitude selon la revendication 1, dans lequel chacune de la pluralité de périodes d'échantillonnage consécutives comprend une pluralité d'intervalles d'échantillonnage d'une minute, et
dans lequel le processeur (106) est configuré en outre pour calculer une période d'inactivité dans chacune de la pluralité de périodes d'échantillonnage consécutives sur la base de données de mouvement inférieures à un niveau d'inactivité prédéterminé.

6. Dispositif de surveillance d'aptitude selon la revendication 5, dans lequel chacune de la pluralité de périodes d'échantillonnage consécutives constitue une période respective d'inactivité et une période respective d'activité, et
dans lequel le processeur (106) est configuré en outre pour calculer la période d'activité dans chacune de la pluralité de périodes d'échantillonnage consécutives comme une agrégation de temps correspondant à des intervalles d'échantillonnage sur lesquels les données de mouvement indiquent que des étapes sont prises par l'utilisateur.

7. Dispositif de surveillance d'aptitude selon la revendication 6, dans lequel :

le processeur (106) est configuré en outre pour classer la période d'activité dans chacune de la pluralité de périodes d'échantillonnage consécutives comme une période d'activité modérée ou une période d'activité intense, la période d'activité modérée correspondent à une agrégation d'intervalles de temps sur lesquels les données de mouvement dépassent un niveau d'inactivité prédéterminé, la période d'activité intense correspondent à une agrégation d'intervalles de temps sur lesquels les données de mouvement dépassent un niveau d'inactivité prédéterminé et un seuil d'activité intense, et le processeur (106) est configuré en outre pour calculer le temps de réinitialisation lorsque chacune de la pluralité de périodes d'échantillonnage consécutives ne comprend aucune période d'activité intense.

8. Un dispositif de surveillance de véhicule comprenant :
un réseau de capteurs (108) configuré pour :

mesurer les mouvements d'un utilisateur portant le dispositif de surveillance de la condition physique (102, 300, 400) et générer des données de mouvement ; et mesurer l'intensité lumineuse et générer des données d'intensité lumineuse ; une mémoire (118) configurée pour stocker des données d'intensité lumineuse et des informations de performance physique ; et un processeur (106) configuré pour:

déterminer les informations de performance physique sur la base des données de mouvement ; suivre les informations de performance physique sur une période de temps prédéterminée ; analyser les données d'intensité lumineuse sur une pluralité d'intervalles d'échantillonnage et une pluralité de périodes d'échantillonnage consécutives, dans lequel chacune des périodes d'échantillonnage est composée d'un multiple des intervalles d'échantillonnage de sorte que les intervalles d'échantillonnage multiples respectifs constituent la période d'échantillonnage respective parmi la pluralité de périodes d'échantillonnage consécutives ;

catégoriser chacun de la pluralité d'intervalles d'échantillonnage sur la base des données d'intensité lumineuse analysées sur chaque intervalle d'échantillonnage respectif comme un intervalle d'échantillonnage ayant une intensité lumineuse mesurée inférieure à un seuil d'intensité lumineuse ou un intervalle d'échantillonnage ayant une intensité lumineuse mesurée supérieure au seuil d'intensité lumineuse ; calculer, à l'aide des données d'intensité lumineuse, un temps de réinitialisation lorsque, au cours de chacune d'une pluralité de périodes d'échantillonnage consécutives, le nombre d'intervalles d'échantillonnage ayant l'intensité lumineuse mesurée en dessous du seuil d'intensité lumineuse dépasse un nombre seuil ; et réinitialiser les informations de performance physique suivies à zéro au moment de la réinitialisation pour recommencer à suivre les informations de performance physique pendant une période approximative d'une journée.

9. Dispositif de surveillance d'aptitude selon la revendication 8, dans lequel le processeur (106) est configuré en outre pour déterminer, en utilisant les données d'intensité lumineuse stockées sur une période de temps prolongée, un temps de minuit approximatif et ajuster le temps de réinitialisation pour qu'il soit égal au temps de minuit approximatif, et/ou dans lequel le processeur (106) est configuré pour calculer le temps de réinitialisation en tant que début de la première de la pluralité de périodes d'échantillonnage consécutives dans lesquelles le nombre d'intervalles d'échantillonnage inactifs dépasse le nombre seuil.

10. Dispositif de surveillance d'aptitude physique selon la revendication 1 ou la revendication 8, dans lequel les informations de performance physique sont le nombre de pas effectués par l'utilisateur, le nombre de pas faisant partie d'un objectif d'activité physique qui représente un nombre seuil de pas à effectuer par l'utilisateur sur la période d'une journée.

11. Dispositif de surveillance d'aptitude selon la revendication dans lequel la période d'échantillonnage est d'une heure.

12. Dispositif de surveillance d'aptitude selon la revendication 8, dans lequel le processeur (106) est configuré pour calculer une chaux de réinitialisation moyenne en utilisant une moyenne mobile de temps de réinitialisation calculée à partir d'une pluralité de périodes de temps et pour mettre à jour la chaux de réinitialisation avec le temps de réinitialisation moyen.

13. Procédé mis en oeuvre par ordinateur dans un dispositif de surveillance d'aptitude (102, 300, 400), comprenant :

générer, à l'aide d'un capteur (108) dans le dispositif de surveillance d'aptitude physique (102, 300, 400), des données de mouvement sur la base de mouvements mesurés d'un utilisateur portant le dispositif de surveillance d'aptitude physique (102, 300, 400) ;

déterminer, à l'aide d'un processeur (106) dans le dispositif de surveillance d'aptitude (102, 300, 400), des informations de performance physique sur la base des données de mouvement ;

suivre, à l'aide du processeur (106), des informations de performance physique sur une période de temps prédéterminée ;

analyser, à l'aide du processeur (106), les données de mouvement sur une pluralité d'intervalles d'échantillonnage et une pluralité de périodes d'échantillonnage consécutives, dans lequel chacune des périodes d'échantillonnage est constituée d'un multiple des intervalles d'échantillonnage de sorte que les intervalles d'échantillonnage multiples respectifs constituent la période d'échantillonnage respective parmi la pluralité de périodes d'échantillonnage consécutives ;

catégoriser, en utilisant le processeur (106), chacun de la pluralité d'intervalles d'échantillonnage en tant qu'intervalle d'échantillonnage actif ou en tant qu'intervalle d'échantillonnage inactif sur la base des données de mouvement analysées sur chaque intervalle d'échantillonnage respectif ;

calculer, à l'aide du processeur (106), un temps de réinitialisation lorsque, dans chacune de la pluralité de périodes d'échantillonnage consécutives, le nombre d'intervalles d'échantillonnage inactifs dépasse un nombre seuil ; et

réinitialiser, à l'aide du processeur (106), les informations de performance physique suivies à zéro au moment de la réinitialisation pour recommencer à suivre les informations de performance physique pendant une période approximative d'une journée.

14. Procédé mis en oeuvre par ordinateur selon la revendication 13, comprenant en outre la détermination, en utilisant les données de mouvement stockées sur une période de temps étendue, d'un temps de minuit approximatif et le réglage du temps de réinitialisation pour qu'il soit égal au temps de minuit approximatif.

15. Procédé mis en oeuvre par ordinateur selon la revendication 13, dans lequel les informations de performance physique sont le nombre de pas effectués par l'utilisateur, le nombre de pas faisant partie d'un objectif d'activité physique qui représente un nombre seuil de pas à effectuer par l'utilisateur sur une période d'une journée ; et dans lequel chacune de la pluralité de périodes d'échantillonnage est d'une heure, chacun de la pluralité d'intervalles d'échantillonnage est d'une minute et la période de temps étendue est de quatre jours,

dans lequel la catégorisation de préférence de chacun de la pluralité d'intervalles d'échantillonnage comprend le processeur (106) :

catégoriser un intervalle d'échantillonnage comme un intervalle inactif lorsque les données de mouvement analysées sur l'intervalle d'échantillonnage indiquent qu'aucun pas n'a été fait par l'utilisateur ; et

catégoriser un intervalle d'échantillonnage comme un intervalle actif lorsque les données de mouvement analysées sur l'intervalle d'échantillonnage indiquent que des pas ont été faits par l'utilisateur.

**FIG. 1**

EP 3 161 779 B1

**FIG. 2A**

**FIG. 2B**

**FIG. 3**

**FIG. 4**

500

ANALYZE MOTION DATA OVER A SAMPLING INTERVAL — 502

CATEGORIZE THE SAMPLING INTERVAL AS AN ACTIVE OR AN INACTIVE SAMPLING INTERVAL — 504

506
HAS AN ENTIRE SAMPLING PERIOD BEEN ANALYZED?

NO

YES

508
ARE THE NUMBER OF INACTIVE SAMPLING INTERVALS GREATER THAN A THRESHOLD?

NO

YES

STORE TIME CORRESPONDING TO SAMPLING PERIOD — 510

512
HAVE A THRESHOLD NUMBER OF SAMPLING PERIODS BEEN STORED?

NO

YES

514
CALCULATE A RESET TIME USING THE STORED SAMPLING PERIOD TIMES

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013069002 A1
- US 2010079294 A1
- WO 2006003243 A1
- US 2012143095 A1